# EUROPÄISCHE PATENTSCHRIFT

(11) **EP 1 353 948 B1**
(45) Veröffentlichungstag und Bekanntmachung des Hinweises auf die Patenterteilung: **19.10.2005**
(21) Anmeldenummer: 01990584.3
(22) Anmeldetag: 21.12.2001
(51) Int. Cl.: C07K 14/47

(54) **LÖSLICHE CYCLISCHE ANALOGA DES BETA AMYLOIDEN PEPTIDS**
SOLUBLE CYCLIC ANALOGUES OF THE BETA AMYLOIDE PEPTIDE
ANALOGUES CYCLIQUES SOLUBLES DU PEPTIDE BETA AMYLOIDE

(30) Priorität: 13.01.2001 DE 10101430
(43) Veröffentlichungstag der Anmeldung: 22.10.2003
(73) Patentinhaber: Fraunhofer-Gesellschaft zur Förderung der angewandten Forschung e.V., 80686 München (DE)
(72) Erfinder: KAPURNIOTU, Afroditi, 62066 Aachen (DE); BERNHAGEN, Jürgen, 62066 Aachen (DE); BRUNNER, Herwig, 70569 Stuttgart (DE)
(86) Internationale Anmeldenummer: PCT/EP2001/015181
(87) Internationale Veröffentlichungsnummer: WO 2002/055552

(56) Entgegenhaltungen:
- WO-A-01/34631
- FINDEIS, MARK A. ET AL: "Modified - Peptide Inhibitors of Amyloid.beta.-Peptide Polymerization" BIOCHEMISTRY (1999), 38(21), 6791-6800 , XP002143947
- HARKANY, TIBOR ET AL: "Neuroprotective approaches in experimental models of beta-amyloid neurotoxicity: relevance to Alzheimer's disease" PROGRESS IN NEURO-PSYCHOPHARMACOLOGY & BIOLOGICAL PSYCHIATRY (1999), 23(6), 963-1008 , XP002210191

### SEQUENZPROTOKOLL

<110> Fraunhofer Ges. zur Förd. der angewandten...
<120> Lösliche cyclische Analoga....
<130> 16069
<140>
   <141>
<160> 19
<170> PatentIn Ver. 2.1
<210> 1
   <211> 43
   <212> PRT
   <213> Homo sapiens
<400> 1
   Asp Ala Glu Phe Arg His Asp Ser Gly Tyr Glu Val His His Gln Lys
    1               5                  10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40
<210> 2
   <211> 28
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 2
   Asp Ala Glu Phe Arg His Asp Ser Gly Tyr Glu Val His His Gln Lys
    1               5                  10                  15
   Lys Val Phe Phe Asp Glu Asp Val Gly Ser Asn Lys
               20                  25
<210> 3
   <211> 40
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 3
   Asp Ala Glu Phe Arg His Asp Ser Gly Tyr Glu Val His His Gln Lys
    1               5                  10                  15
   Lys Val Phe Phe Asp Glu Asp Val Gly Ser Asn Lys Gly Ala Ile Ile
               20                  25                  30
   Gly Leu Met Val Gly Gly Val Val
           35                  40
<210> 4
   <211> 42
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 4
   Asp Ala Glu Phe Arg His Asp Ser Gly Tyr Glu Val His His Gln Lys
    1               5                  10                  15
   Lys Val Phe Phe Asp Glu Asp Val Gly Ser Asn Lys Gly Ala Ile Ile
               20                  25                  30
   Gly Leu Met Val Gly Gly Val Val Ile Ala
           35                  40
<210> 5
   <211> 28
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 5
   Asp Ala Glu Phe Arg His Asp Ser Gly Tyr Glu Val His His Gln Lys
   1               5                   10                  15
   Asp Val Phe Phe Lys Glu Asp Val Gly Ser Asn Lys
               20                  25
<210> 6
   <211> 40
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 6
   Asp Ala Glu Phe Arg His Asp Ser Gly Tyr Glu Val His His Gln Lys
   1               5                   10                  15
   Asp Val Phe Phe Lys Glu Asp Val Gly Ser Asn Lys Gly Ala Ile Ile
               20                  25                  30
   Gly Leu Met Val Gly Gly Val Val
           35                  40
<210> 7
   <211> 42
   <212> PRT
   <213> Homo sapiens
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 7
   Asp Ala Glu Phe Arg His Asp Ser Gly Tyr Glu Val His His Gln Lys
    1               5                  10                  15
   Asp Val Phe Phe Lys Glu Asp Val Gly Ser Asn Lys Gly Ala Ile Ile
               20                  25                  30
   Gly Leu Met Val Gly Gly Val Val Ile Ala
           35                  40
<210> 8
   <211> 28
   <212> PRT
   <213> Homo sapiens
<220>
   <221> PEPTIDE
   <222> (21)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 8
   Asp Ala Glu Phe Arg His Asp Ser Gly Tyr Glu Val His His Gln Lys
    1               5                  10                  15
   Asp Val Phe Phe Xaa Glu Asp Val Gly Ser Asn Lys
               20                  25
<210> 9
   <211> 40
   <212> PRT
   <213> Homo sapiens
<220>
   <221> PEPTIDE
   <222> (21)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 9
   Asp Ala Glu Phe Arg His Asp Ser Gly Tyr Glu Val His His Gln Lys
   1 5 10 15
   Asp Val Phe Phe Xaa Glu Asp Val Gly Ser Asn Lys Gly Ala Ile Ile
   20 25 30
   Gly Leu Met Val Gly Gly Val Val
   35 40
<210> 10
   <211> 42
   <212> PRT
   <213> Homo sapiens
<220>
   <221> PEPTIDE
   <222> (21)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 10
   Asp Ala Glu Phe Arg His Asp Ser Gly Tyr Glu Val His His Gln Lys
   1 5 10 15
   Asp Val Phe Phe Xaa Glu Asp Val Gly Ser Asn Lys Gly Ala Ile Ile
   20 25 30
   Gly Leu Met Val Gly Gly Val Val Ile Ala
   35 40
<210> 11
   <211> 28
   <212> PRT
   <213> Homo sapiens
<220>
   <221> PEPTIDE
   <222> (21)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 11
   Asp Ala Glu Phe Arg His Asp Ser Gly Tyr Glu Val His His Gln Lys
   1               5                   10                  15
   Asp Val Phe Phe Xaa Glu Asp Val Gly Ser Asn Lys
               20                  25
<210> 12
   <211> 40
   <212> PRT
   <213> Homo sapiens
<220>
   <221> PEPTIDE
   <222> (21)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 12
   Asp Ala Glu Phe Arg His Asp Ser Gly Tyr Glu Val His His Gln Lys
   1               5                   10                  15
   Asp Val Phe Phe Xaa Glu Asp Val Gly Ser Asn Lys Gly Ala Ile Ile
               20                  25                  30
   Gly Leu Met Val Gly Gly Val Val
           35                  40
<210> 13
   <211> 42
   <212> PRT
   <213> Homo sapiens
<220>
   <221> PEPTIDE
   <222> (21)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 13
   Asp Ala Glu Phe Arg His Asp Ser Gly Tyr Glu Val His His Gln Lys
   1 5 10 15
   Asp Val Phe Phe Xaa Glu Asp Val Gly Ser Asn Lys Gly Ala Ile Ile
   20 25 30
   Gly Leu Met Val Gly Gly Val Val Ile Ala
   35 40
<210> 14
   <211> 28
   <212> PRT
   <213> Homo sapiens
<220>
   <221> PEPTIDE
   <222> (17)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 14
   Asp Ala Glu Phe Arg His Asp Ser Gly Tyr Glu Val His His Gln Lys
   1 5 10 15
   Xaa Val Phe Phe Asp Glu Asp Val Gly Ser Asn Lys
   20 25
<210> 15
   <211> 40
   <212> PRT
   <213> Homo sapiens
<220>
   <221> PEPTIDE
   <222> (17)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbünden.
<400> 15
   Asp Ala Glu Phe Arg His Asp Ser Gly Tyr Glu Val His His Gln Lys
   1 5 10 15
   Xaa Val Phe Phe Asp Glu Asp Val Gly Ser Asn Lys Gly Ala Ile Ile
   20 25 30
   Gly Leu Met Val Gly Gly Val Val
   35 40
<210> 16
   <211> 42
   <212> PRT
   <213> Homo sapiens
<220>
   <221> PEPTIDE
   <222> (17)
   <223> Xaa=Orn
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 16
   Asp Ala Glu Phe Arg His Asp Ser Gly Tyr Glu Val His His Gln Lys
   1 5 10 15
   Xaa Val Phe Phe Asp Glu Asp Val Gly Ser Asn Lys Gly Ala Ile Ile
   20 25 30
   Gly Leu Met Val Gly Gly Val Val Ile Ala
   35 40
<210> 17
   <211> 28
   <212> PRT
   <213> Homo sapiens
<220>
   <221> PEPTIDE
   <222> (17)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 17
   Asp Ala Glu Phe Arg His Asp Ser Gly Tyr Glu Val His His Gln Lys
   1 5 10 15
   Xaa Val Phe Phe Asp Glu Asp Val Gly Ser Asn Lys
   20 25
<210> 18
   <211> 40
   <212> PRT
   <213> Homo sapiens
<220>
   <221> PEPTIDE
   <222> (17)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 18
   Asp Ala Glu Phe Arg His Asp Ser Gly Tyr Glu Val His His Gln Lys
   1 5 10 15
   Xaa Val Phe Phe Asp Glu Asp Val Gly Ser Asn Lys Gly Ala Ile Ile
   20 25 30
   Gly Leu Met Val Gly Gly Val Val
   35 40
<210> 19
   <211> 42
   <212> PRT
   <213> Homo sapiens
<220>
   <221> PEPTIDE
   <222> (17)
   <223> Xaa=Dab
<220>
   <221> PEPTIDE
   <222> (17)..(21)
   <223> Die Seitenketten der Aminosäuren an den Positionen 17 und 21 sind über eine intramolekulare Brücke miteinander verbunden.
<400> 19
   Asp Ala Glu Phe Arg His Asp Ser Gly Tyr Glu Val His His Gln Lys
    1               5                  10                  15
   Xaa Val Phe Phe Asp Glu Asp Val Gly Ser Asn Lys Gly Ala Ile Ile
               20                  25                  30
   Gly Leu Met Val Gly Gly Val Val Ile Ala
           35                  40

## Beschreibung

Die vorliegende Erfindung betrifft Peptide mit der biologischen Aktivität eines Modulators der Amyloidogenese, Verfahren zum Auffinden amyloidogener Peptide oder deren Aggregate, pharmazeutische Zusammensetzungen zur Prophylaxe und Therapie mit Amyloidogenese einhergehender Krankheiten sowie diagnostische Zusammensetzungen zum Nachweis derartiger Krankheiten.

Amyloide Krankheiten oder Amyloidosen sind Krankheiten, die mit einer Amyloidogenese, Amyloidbildung beziehungsweise Aggregation amyloider Proteine in bestimmten Körpergeweben einhergehen, womit häufig cytotoxische Effekte verbunden sind. So geht die Alzheimersche Krankheit mit einer Amyloidogenese des β-amyloiden Peptids (β-AP) einher.

Es wird angenommen, dass die Bildung der Amyloidablagerung verschiedener Peptide verantwortlich ist für das Entstehen und die pathologische Komplikationen amyloider Krankheiten (Lorenzo et al., Nature 368 (1994), 756-760, Lorenzo et al., Proc. Natl. Acad. Sci. USA 91 (1994), 12243-12247, Lansbury et al., Proc. Natl. Acad. Sci. USA 96 (1999)., 3342-3344, Koo et al., Proc. Natl. Acad. Sci. USA 96 (1999), 9989-9990, Sipe et al., Critical Reviews in Clinical Laboratory Sciences 31(4) (1994), 325-354). Es wird ferner angenommen, dass Inhibitoren der Amyloidbildung geeignete Pharmazeutika zur Prophylaxe und Bekämpfung der amyloiden Krankheiten darstellen.

Aus der DE-A1 197 25 619 sind lineare Peptide aus 3 bis 15 Aminosäuren bekannt, die als Agonisten und/oder Inhibitoren der Amyloidbildung wirken können. Diese Peptide zeichnen sich dadurch aus, dass sie als wirksame Sequenz mindestens die Aminosäuren GA enthalten.

Aus dem US-Patent 5,854,204 sind Peptide bekannt, die die β-Amyloidaggregation modulieren. Es handelt sich um linear aufgebaute Peptide unterschiedlicher Länge, welche insbesondere von dem β-Amyloidvorläuferprotein 770 abgeleitet ist.

Die WO 96/39834 beschreibt weitere lineare Peptide, die in der Lage sind, Krankheiten mit abnormaler Peptidzusammenlagerung zu heilen. Die beschriebenen Peptide weisen 3 bis 15 Aminosäuren, einen hydrophoben Abschnitt von 3 Aminosäuren und in diesem hydrophoben Abschnitt mindestens eine eine β-Faltblattstruktur blockierende Aminosäure auf.

Die WO 96/07425 beschreibt neurotoxische Effekte des Amyloid-β-Proteins durch inhibierende synthetische Peptide mit linearer Struktur. Die Diagnose und Behandlung von Typ II Diabetes mellitus unter Verwendung von Amylin und Derivaten davon ist in der EP 0 289 287 und der EP 0 309 100 A2 beschrieben.

Aus der WO 01/34631 A2 ist ein cyclisiertes Pentapeptid bekannt, welches ein Fragment des β-amyloiden Peptids darstellt, und die Eigenschaft besitzt, die Tendenz zur Bildung der β-Faltblattstruktur zu hemmen. Durch Austausch bestimmter Aminosäuren durch Cystein wird die Cyclisierung durch die Bildung von Disulfid-Brücken ermöglicht.

Aus der Publikation von Findeis et al. (Biochemistry 1999, 38:6791-6800) sind (lineare) Peptide bekannt, die als Inhibitoren der Polymerisation des Amyloid-β-Proteins dienen. Diese Peptide wurden entweder neu syntetisiert oder durch eine Reihe verschiedener Modifikationsschritte von der nativen Form des β-amyloiden Proteins abgeleitet. Die Modifikationen umfassen unter anderem selektive Deletion, Modifikation mit organischen Reagenzien, Verkürzung zu einem Pentadekapaptid mit N-terminaler Modifikation sowie Cholyl-Modifikationen.

Testverfahren zur Früherkennung der amyloiden Krankheiten sind zur Zeit noch nicht verfügbar beziehungsweise erst im Entwicklungsstadium. Dies liegt daran, dass die durch die Amyloidbildung verursachten proteinchemischen und technischanalytischen Probleme bisher keine Analytik der Amyloidbildung erlauben. Als Folge davon ist der Mechanismus der Amyloidbildung im Wesentlichen unaufgeklärt. Als weitere Folge davon existieren gegenwärtig keine pharmazeutischen Ansätze zur Behandlung der amyloiden Krankheiten auf der Basis von Modulatoren, insbesondere Inhibitoren, der Amyloidogenese. Ebenso wenig existieren auf der Amyloidbildungsanalyse aufbauende diagnostische Verfahren, zum Beispiel in vitro-Tests zur Bewertung von Kinetik, Quantität und Qualität der Bildung amyloider Strukturen. Nach wie vor muss die Diagnose, beispielsweise der Alzheimerschen Krankheit, symptomatisch durchgeführt werden.

Insbesondere besteht ein Bedarf an geeigneten Sonden, die in der Lage sind, amyloidbildende Peptide nachzuweisen, um so schon im Vorfeld möglicher Krankheiten abnormale Erhöhungen der Konzentration amyloidogener Peptide festzustellen. Die Bereitstellung derartiger Sonden scheiterte bisher daran, dass Sonden selbst nicht aggregieren dürfen, gleichwohl aber spezifisch mit den amyloidogenen Peptiden interagieren, das heißt binden, müssen. Ähnlich sind die Anforderungen, welche an einen Inhibitor der Amyloidbildung gestellt werden. Auch ein solcher Stoff darf selbstverständlich die Aggregation nicht fördern beziehungsweise selber aggregieren, und zudem sollte er spezifisch die amyloidogenen Peptide binden können, um ihre weitere Aggregation zu unterbinden. Eine den vorgenannten Kriterien gerecht werdende Substanz konnte bisher noch nicht erfolgreich in der medizinischen Diagnostik und Therapie eingesetzt werden. Es besteht daher weiterhin ein großer Bedarf an der Bereitstellung von Substanzen, die sowohl als Sonden für die Diagnose, insbesondere Früherkennung, von mit einer Amyloidbildung einhergehenden Krankheiten als auch für die Therapie einschließlich Prophylaxe derselben dienen können.

Der vorliegenden Erfindung liegt daher das technische Problem zugrunde, Substanzen bereitzustellen, die in der Lage sind, als Sonden für die Diagnose mit einer Amyloidbildung einhergehender Krankheiten, insbesondere der Alzheimerschen Krankheit, sowie der Therapie derselben dienen können.

Die vorliegende Erfindung löst das ihr zugrunde liegende technische Problem durch die Bereitstellung von Peptiden mit der biologischen Aktivität eines Modulators der Amyloidogenese, insbesondere eines Inhibitors der Amyloidogenese, wobei das Peptid eine Aminosäuresequenz, ausgewählt aus der Gruppe der Aminosäuresequenzen bestehend aus SEQ ID Nr. 1 bis SEQ ID Nr. 19 gemäß beiliegendem Sequenzprotokoll, aufweist und zumindest die Aminosäuren des Peptids an Position 17 und 21 über ihre Seitenketten eine intramolekulare Brücke mittels kovalenter Bindung bilden. Dabei kann vorgesehen sein, dass die Brücke durch direkte Bindung zwischen funktionellen Gruppen, zum Beispiel Amino- und Carboxygruppen, der Seitenketten erfolgt. Es kann aber auch vorgesehen sein, dass die Seitenketten der an der Brückenbildung beteiligten Aminosäuren des Peptides über einen Spacer oder Linker, das heißt ein Verbindungsmolekül, beispielsweise eine Aminocarboxy-, Diamino- oder Dicarboxyverbindung, miteinander kovalent verbunden sind.

Die Aggregation und krankheitsverursachende Amyloidbildung findet durch eine intermolekulare β-Faltblatt-Bildung zwischen den amyloidogenen Peptidsequenzen statt, wofür die Ausbildung intermolekularer Wasserstoffbrücken und hydrophobe Wechselwirkungen zwischen den Seitenketten bestimmter Aminosäurereste notwendig ist. Diese β-Faltblatt-Struktur führt zu einer nicht-kovalenten Bindung zwischen zunächst zwei und anschließend mehreren amyloidogenen Peptidketten, also β-AP-Molekülen, welche daraufhin unlösliche Aggregate beziehungsweise Amyloid-Strukturen bilden.

Die erfindungsgemäß vorgesehene intramolekulare Brücke in β-AP mit der biologischen Aktivität eines Modulators der Amyloidogenese verhindert, ohne an die Theorie gebunden zu sein, den Übergang zwischen einer ungeordneten, α-helikalen Konformation oder einem nicht amyloidogenen Zustand in eine aggregierte bzw. aggregationsfähige β-Faltblattstruktur. Erfindungsgemäß wird durch das Einführen der intramolekularen Brücke eine Konformationsrestriktion, das heißt Stabilisierung eines bestimmten nicht amyloidogenen Konformationszustandes, zum Beispiel β-Turn oder α-Helix, erzielt. Die erfindungsgemäßen cyclischen Peptide sind daher hinsichtlich ihrer Primärstruktur sehr ähnlich zu den Wildtypmolekülen, als Wildtyp-β-AP, wobei jedoch starke Unterschiede in Bezug auf die Sekundärstruktur zwischen diesen Peptiden und den nativen Peptiden vorhanden sind. Die erfindungsgemäß erzielte Konformationsrestriktion von β-AP in einer nicht aggregationsfähigen Konformation stellt demgemäss nicht amyloidogene Moleküle zur Verfügung, die als Aggregationsinhibitoren oder Amyloidbildungsinhibitoren fungieren und demgemäss als lösliche Agonisten und Sonden von beziehungsweise für amyloide Peptide wirken können. Die erfindungsgemäßen cyclischen Peptide zeichnen sich nämlich dadurch aus, dass sie einerseits spezifisch an die native, das heißt Wildtypform, des ihrer Struktur zugrunde liegenden amyloiden Peptids nicht kovalent binden oder sich mit ihr assoziieren können und so in der Lage sind, diese zu detektieren. Die erfindungsgemäßen Peptide zeichnen sich darüber hinaus dadurch aus, dass sie selber nicht amyloidogen sind und gleichzeitig bei Bindung an die native Form des Peptids dessen Assoziation mit weiteren nativen Peptiden inhibieren. Insbesondere findet eine Bindung der erfindungsgemäßen cyclischen Peptide an noch nicht aggregierte lösliche Formen beziehungsweise an noch lösliche Mono- und Oligomere des nativen Peptides statt.

Die erfindungsgemäßen cyclischen Peptide wirken also als Agonisten, Sonden und Amyloidbildungsinhibitoren, die für die Diagnostik und Therapie von mit Amyloidbildung einhergehenden Krankheiten eingesetzt werden können. Darüber hinaus können sie als molekulares Werkzeug zur Analyse und Untersuchung der Amyloidogenese sowie zur Erforschung, Entwicklung und Herstellung von auf die Alzheimersche Krankheit bezogenen Diagnostika und Therapeutika sowie zum Schutz vor der Alzheimerschen Krankheit auch als Impfstoffe eingesetzt werden. Durch die erfindungsgemäß erzielte Inhibition der Amyloidogenese der krankheitsverursachenden Peptide wird gleichzeitig die zytotoxische Wirkung der Amyloidaggregate auf Gewebszellen inhibiert und ein weiterer wichtiger Krankheitsauslöser ausgeschaltet. Die erfindungsgemäßen cyclischen Peptide lassen sich durch eine Chemosynthese in hoher Reinheit nach gängigen Methoden der Festphasenpeptidsynthese herstellen. Sie weisen eine hohe biologische, insbesondere proteolytische, Stabilität auf, welche beispielsweise durch den Einbau von unnatürlichen Aminosäuren und Ringstrukturen erreicht beziehungsweise weiter erhöht werden kann. Die erfindungsgemäßen cyclischen Peptide sind einfach handhabbar, was unter anderem auf ihrer hohen Löslichkeit beruht. Dies steht im Gegensatz zu der problematischen Handhabbarkeit der nativen Moleküle, also nativem β-AP. Die erfindungsgemäßen cyclischen Peptide weisen ferner, wenn überhaupt, nur geringe Nebenwirkungen und Antigenizität beim Einsatz als Therapeutikum auf, da die erfindungsgemäßen Amyloidbildungsinhibitoren eine sehr ähnliche Primärstruktur wie die im Körper vorkommenden nativen Peptide aufweisen.

Im Zusammenhang mit der vorliegenden Erfindung wird unter einem Peptid ein mindestens zwei über eine Amidbindung miteinander verknüpfte Aminosäuren aufweisendes Molekül verstanden, zum Beispiel ein Oligopeptid, ein Polypeptid oder ein Protein. Das Peptid kann ein natürlicherweise vorkommendes Peptid, eine chemisch modifizierte Variante davon oder ein denovo synthetisiertes Peptid sein. Das Peptid kann Modifizierungen aufweisen, wie beispielsweise Glycosylierungen oder andere Derivatisierungen wie Alkylierungen, Hydroxylierungen, Aminierungen oder ähnliches. Das Peptid kann gegenüber der natürlicherweise vorkommenden Form, also der nativen Form, abgewandelt sein, also beispielsweise ein strukturelles und/oder funktionelles Äquivalent darstellen wie ein Peptidanalog oder ein Peptidomimetikum, wobei diese Abwandlungen in Aminosäureinsertionen, Aminosäureaustauschen, Aminosäureinversionen, Aminosäureadditionen oder/und Aminosäuredeletionen bestehen können. Das Peptid kann auch ungewöhnliche und/oder unnatürliche Aminosäuren enthalten. Ebenso können die Aminosäuren des Peptids in D- oder L-Konfiguration vorliegen.

Im Zusammenhang mit der vorliegenden Erfindung wird unter einem Peptid mit der biologischen Aktivität eines Modulators der Amyloidogenese ein Peptid verstanden, welches in der Lage ist, Amyloid- oder A-myloidähnliche Strukturen bildende Peptide hinsichtlich ihrer Aggregationsfähigkeit zu Amyloiden zu beeinflussen, insbesondere die Amyloidogenese zu fördern, zu initiieren oder zu inhibieren, zum Beispiel zu reduzieren oder zu blockieren. Eine Förderung der Aggregationsfähigkeit kann für Forschungsarbeiten oder für die gezielte, gewebespezifische Lokalisation von Amyloiden in Geweben, in denen sie nicht schädlich sind, erwünscht sein. Ein Peptid mit der biologischen Aktivität eines Modulators der Amyloidogenese zeichnet sich insbesondere dadurch aus, dass dieses Peptid in der Lage ist, mit amyloidogenen Peptiden derart zu interagieren, dass deren Aggregationsfähigkeit, insbesondere hinsichtlich der Aggregationsrate, also der Aggregationsgeschwindigkeit, und der Größe bzw. Menge gebildete Aggregate, geändert, insbesondere inhibiert wird. Diese Änderung der Aggregationsfähigkeit amyloidogener Peptide findet vorzugsweise durch Assoziation beziehungsweise Anheften, vorzugsweise durch nichtkovalente Bindung, an die vorzugsweise lösliche Form des amyloidogenen Peptids oder eines löslichen Oligomers des amyloidogenen Peptides statt. In besonders bevorzugter Ausführungsform ist ein inhibitorischer Effekt der vorliegenden Erfindung ein Effekt, gemäss dem die lag-Phase der Aggregation wenigstens 1,5; 1,8; 2; 2,5; 3; 4; 5; 8; 10; 20; 40; 50; oder 100-fach gegenüber der mit nur natürlicherweise vorkommenden amyloidogenen Peptiden vorliegenden lag-Phase verlängert ist. In einer weiteren Ausführungsform der Erfindung liegt ein inhibitorischer Effekt dann vor, wenn der Aggregationsgrad gegenüber dem bei nur natürlicherweise vorkommenden amyloidogenen Peptiden beziehungsweise daraus gebildeten Aggregaten vorhandenen Aggregationsgrad um 10, 20, 30, 40, 50, 60, 70, 80, 90 oder 100 % vermindert ist.

Im Zusammenhang mit der vorliegenden Erfindung wird unter dem Begriff β-AP ein Peptid mit der biologischen Aktivität eines Modulators der Amyloidogenese verstanden, wobei dieses eine intramolekulare Brücke aufweist und gegenüber der Wildtypsequenz eine abweichende Primärstruktur, das heißt Aminiosäuresequenz aufweisen kann. Zur Ermittlung der Modulation der Amyloidogenese, der Aggregationsrate, dem Aggregationsgrad (Gesamtmenge an Aggregation) und der Aggregationsgeschwindigkeit sowie -fähigkeit können üblicherweise bekannte Testverfahren, wie sie beispielsweise in der US 5,854,204 beschrieben sind, eingesetzt werden, die im Zusammenhang mit den dort beschriebenen Testverfahren in den Offenbarungsgehalt der vorliegenden Lehre mit einbezogen werden.

Im Zusammenhang mit der vorliegenden Erfindung werden unter dem Begriff "an der Brückenbildung beteiligte Aminosäuren" diejenigen Aminosäuren des Peptides verstanden, deren Seitenketten die funktionellen Gruppen aufweisen, die zu einer kovalenten, zu der intramolekularen Brücke führenden Bindung direkt oder indirekt miteinander verknüpft sind. Die an der Brückenbildung beteiligten Aminosäuren weisen also Seitenketten auf, die funktionelle Gruppen umfassen, die entweder jeweils direkt mit anderen funktionellen Gruppen einer anderen Seitenkette einer anderen Aminosäure des Peptides verknüpft sind oder deren funktionelle Gruppe mit mindestens einem Spacermolekül verknüpft sind, welches wiederum mit der funktionellen Gruppe einer Seitenkette einer anderen an der Brückenbildung beteiligten Aminosäure des Peptids verknüpft ist. Derartige funktionelle Gruppen können zum Beispiel Hydroxy-, Amino-, Carboxyl-, und/oder Thiolgruppen sein. Die an der Brückenbildung beteiligten Aminosäuren können natürlicherweise in der nativen Sequenz an dieser Position vorhandene Aminosäuren sein. Erfindungsgemäß ist es auch möglich, durch geeignete Substitutionen der nativ vorkommenden Aminosäuren durch an dieser Stelle natürlicherweise nicht vorkommende oder unnatürliche oder ungewöhnliche Aminosäuren substituierende Aminosäuren einzuführen, die für die Brückenbildung verwendet werden beziehungsweise daran beteiligt sind. Selbstverständlich ist es auch möglich, dass die an der Brückenbildung beteiligten Aminosäuren zusätzlich in die Aminosäuresequenz des β-AP eingefügt wurden, ohne dass eine natürlicherweise vorkommende Aminosäure deletiert wird. Das Einführen natürlicherweise an dieser Position nicht vorkommender Aminosäuren kann den Vorteil aufweisen, dass gezielt an dieser Position spezifische funktionelle Gruppen eingeführt werden, die eine Brückenbildung ermöglichen oder erleichtern. Erfindungsgemäß ist es vorgesehen, als an der Brückenbildung beteiligte Aminosäuren Dab (Dab ist 2,4-Diaminobuttersäure), Dap (Dap ist 2,3 Diaminopropionsäure), Ser, Asp, Glu, Cys, Lys und/oder Orn in die Aminosäuresequenz einzuführen, und zwar als Substitution.

Die Erfindung sieht in einer vorteilhaften Ausgestaltung vor, dass an der Brückenbildung beteiligte Aminosäuren des Peptides im Abstand von i+3, i+4, i+5, i+6 oder i+7 zueinander in dem Peptid angeordnet sind.

In einer bevorzugten Ausführungsform der vorliegenden Erfindung wird vorgesehen, die Seitenketten der an der Brückenbildung beteiligten Aminosäuren des Peptides über einen Spacer miteinander zu verknüpfen, wobei der Spacer ausgewählt ist, aus der Gruppe bestehend aus X-(CH₂)ₙ-Y mit n = 1 bis 6; und X/Y: NH₂/COOH oder COOH/NH₂ oder NH₂/NH₂ oder COOH/COOH oder OH/OH oder SH/SH; oder X ist eins von NH₂ oder COOH oder OH oder SH und Y ist eins von COOH oder NH₂ oder SH oder OH. Selbstverständlich ist es möglich, auch andere Spacer einzusetzen. Erfindungsgemäß von Bedeutung ist, dass die Natur von X und Y, also den endständigen funktionellen Gruppen des Spacers oder Linkers an die funktionellen Gruppen der zu verknüpfenden Seitenketten so angepasst ist, dass eine chemisch kovalente Bindung eingegangen werden kann, beispielsweise durch Verknüpfung einer Aminogruppe mit einer Carboxygruppe oder einer Carboxygruppe mit einer Hydroxylgruppe oder zweier Hydroxylgruppen oder zweier SH-Gruppen.

In einer weiteren bevorzugten Ausführungsform können die cyclischen Peptide der vorliegenden Erfindung weitere funktionelle Gruppen aufweisen, die nicht im Zusammenhang mit der Brückenbildung stehen, sondern beispielsweise der Immobilisierung an Trägern, der Wechselwirkung mit anderen Molekülen, der Detektion oder Ähnlichem dienen. Derartige Gruppen können funktionalisierte Alkylgruppen, aromatische Gruppen, Glykogruppen, Lipidgruppen, zyklische-, heterozyklische- oder polyzyklische Gruppen, biotinhaltige Gruppen, Avidin-haltige Gruppen, Streptavidin-haltige Gruppen oder Ähnliches sein. Selbstverständlich kann auch vorgesehen sein, das Peptid der vorliegenden Erfindung mit anderen Peptiden, Polypeptiden oder Proteinen oder Fragmenten davon zu fusionieren, zum Beispiel mit Wildtyp-β-AP, Derivaten oder Fragmenten davon.

Die erfindungsgemäßen Peptide können Markierungen aufweisen, zum Beispiel Enzyme, prosthetische Gruppen, fluoreszente Materialien, radioaktive Materialien oder lumineszente Materialien.

Als Enzym kann beispielsweise die Meerrettichperoxidase, die alkalische Phosphatase, die β-Galaktosidase oder die Acetylcholinesterase eingesetzt werden. Als prostethische Gruppen können beispielsweise Streptavidin/Biotin, Avidin/Biotin und als fluoreszente Materialien Umbelliferon, Fluorescein, Fluorescein-Isothiocyanat, Rhodamin, Dichlorotriazinylaminfluorescein, Dansylchlorid oder Phycoerythrin eingesetzt werden. Als radioaktive Materialien können beispielsweise ¹⁴C, ¹²³I, ¹²⁴I, ¹²⁵I, ¹³¹I, ^{99m}Tc, ³⁵S oder ³H eingesetzt werden.

Die in der vorliegenden Lehre verwendeten Aminosäurenummerierungen und Positionen beziehen sich auf die Sequenz des nativen Wildtyp-β-AP, so wie es in Tabelle 1 von Hilbich et al. (J. Mol. Biol. 228 (1992), 460-473) beschrieben ist. Der Offenbarungsgehalt dieser Druckschrift wird hinsichtlich der Sequenz der Aminosäuren von β-AP sowie dessen Bereitstellung vollinhaltlich in den Offenbarungsgehalt der vorliegenden Lehre mit einbezogen und klargestellt, dass im erfindungsgemäßen Kontext dafür Schutz begehrt wird. Die Nummerierung gemäß der vorliegenden Lehre erfolgt immer vom N-Terminus zum C-Terminus.

In einer weiteren besonders bevorzugten Ausführungsform der vorliegenden Erfindung, gemäß der das ursprüngliche Peptid, also das Stammpeptid, β-AP ist, befinden sich die erfindungsgemäß an der Brückenbildung beteiligten Aminosäuren im Bereich der Positionen 15 bis 24 des β-AP. Gemäß einer weiteren bevorzugten Ausgestaltung dieser auf das β-AP Stammpeptid zurückzuführenden Ausführungsform weist das β-AP 1 bis 28 (β-AP 1-28 oder β-AP128), 1 bis 40 (β-AP 1-40 oder β-AP140), 1 bis 42 (β-AP 1-42 oder β-AP142) oder 1 bis 43 (β-AP 1-43 oder β-AP143) Aminosäuren auf. In einer weiteren bevorzugten Ausführungsform der vorliegenden Erfindung sind die an der Brückenbildung beteiligten Aminosäuren des β-AP die natürlicherweise oder dort zum Beispiel als Substitution eingeführten Aminosäuren, zum Beispiel Lysin und Asparaginsäure, zum Beispiel an den Positionen 17 und 21.

In einer besonders bevorzugten Ausführungsform der vorliegenden Erfindung betrifft die Erfindung das cyclische Peptid Cyclo^{17,21} [Lys¹⁷, Asp²¹] β-AP (1 bis 28)(abgekürzt als: cβ-AP 128 oder cβ-AP (1 bis 28)). Die Aminosäuresequenz ist dargestellt in SEQ ID No. 2.

In einer weiteren bevorzugten Ausführungsform ist das cyclische Peptid Cyclo^{17,21} [Lys¹⁷, Asp²¹] β-AP (1 bis 40)(abgekürzt als: cβ-AP 140). Die Aminosäuresequenz ist dargestellt in SEQ ID No. 3.

In einer weiteren bevorzugten Ausführungsform ist das cyclische Peptid Cyclo^{17,21} [Lys¹⁷, Asp²¹] β-AP (1 bis 42) (abgekürzt als: cβ-AP 142). Die Aminosäuresequenz ist dargestellt in SEQ ID No. 4.

In einer besonders bevorzugten Ausführungsform der vorliegenden Erfindung betrifft die Erfindung das cyclische Peptid Cyclo^{17,21} [Asp¹⁷, Lys²¹] β-AP (1 bis 28). Die Aminosäuresequenz ist dargestellt in SEQ ID No. 5.

In einer weiteren bevorzugten Ausführungsform ist das cyclische Peptid Cyclo^{17,21} [Asp¹⁷, Lys²¹] β-AP (1 bis 40). Die Aminosäuresequenz ist dargestellt in SEQ ID No. 6.

In einer weiteren bevorzugten Ausführungsform ist das cyclische Peptid Cyclo^{17,21} [Asp¹⁷, Lys²¹] β-AP (1 bis 42). Die Aminosäuresequenz ist dargestellt in SEQ ID No. 7.

In einer besonders bevorzugten Ausführungsform der vorliegenden Erfindung betrifft die Erfindung das cyclische Peptid Cyclo^{17,21} [Asp¹⁷, Orn²¹] β-AP (1 bis 28)(Orn: Ornithin) Die Aminosäuresequenz ist dargestellt in SEQ ID No. 8.

In einer weiteren bevorzugten Ausführungsform ist das cyclische Peptid Cyclo^{17,21} [Asp¹⁷, Orn²¹] β-AP (1 bis 40). Die Aminosäuresequenz ist dargestellt in SEQ ID No. 9.

In einer weiteren bevorzugten Ausführungsform ist das cyclische Peptid Cyclo^{17,21} [Asp¹⁷, Orn²¹] β-AP (1 bis 42). Die Aminosäuresequenz ist dargestellt in SEQ ID No. 10.

In einer besonders bevorzugten Ausführungsform der vorliegenden Erfindung betrifft die Erfindung das cyclische Peptid Cyclo^{17,21} [Asp¹⁷, Dab²¹] β-AP (1 bis 28) (Dab: 2,4-Diaminobuttersäure). Die Aminosäuresequenz ist dargestellt in SEQ ID No. 11.

In einer weiteren bevorzugten Ausführungsform ist das cyclische Peptid Cyclo^{17,21} [Asp¹⁷, Dab²¹] β-AP (1 bis 40). Die Aminosäuresequenz ist dargestellt in SEQ ID No. 12.

In einer weiteren bevorzugten Ausführungsform ist das cyclische Peptid Cyclo^{17,21} [Asp¹⁷, Dab²¹] β-AP (1 bis 42). Die Aminosäuresequenz ist dargestellt in SEQ ID No. 13.

In einer besonders bevorzugten Ausführungsform der vorliegenden Erfindung betrifft die Erfindung das cyclische Peptid Cyclo^{17,21} [Orn¹⁷, Asp²¹] β-AP (1 bis 28). Die Aminosäuresequenz ist dargestellt in SEQ ID No. 14.

In einer weiteren bevorzugten Ausführungsform ist das cyclische Peptid Cyclo^{17,21} [Orn¹⁷, Asp²¹] β-AP (1 bis 40). Die Aminosäuresequenz ist dargestellt in SEQ ID No. 15.

In einer weiteren bevorzugten Ausführungsform ist das cyclische Peptid Cyclo^{17,21} [Orn¹⁷, Asp²¹] β-AP (1 bis 42). Die Aminosäuresequenz ist dargestellt in SEQ ID No. 16.

In einer besonders bevorzugten Ausführungsform der vorliegenden Erfindung betrifft die Erfindung das cyclische Peptid Cyclo^{17,21} [Dab¹⁷, Asp²¹] β-AP (1 bis 28). Die Aminosäuresequenz ist dargestellt in SEQ ID No. 17.

In einer weiteren bevorzugten Ausführungsform ist das cyclische Peptid Cyclo^{17,21} [Dab¹⁷, Asp²¹] β-AP (1 bis 40). Die Aminosäuresequenz ist dargestellt in SEQ ID No. 18.

In einer weiteren bevorzugten Ausführungsform ist das cyclische Peptid Cyclo^{17,21} [Dab¹⁷, Asp²¹] β-AP (1 bis 42). Die Aminosäuresequenz ist dargestellt in SEQ ID No. 19.

Die Erfindung betrifft auch Antikörper, insbesondere monoclonale oder polyclonale Antikörper, die spezifisch die cyclischen Peptide der Erfindung erkennen und an diese binden können. Die Antikörper können in üblicher Weise modifiziert, zum Beispiel markiert sein. Sie können auch in immobilisierter Form auf einem Träger oder an Kügelchen fixiert vorliegen.

Die Erfindung sieht auch Verfahren vor, gemäß derer die erfindungsgemäßen Peptide als Antigene zur Immunisierung von menschlichen oder tierischen Organismen verwendet werden und die gebildeten Antikörper gewonnen werden. Die Erfindung betrifft also auch Verfahren zur Gewinnung von mono- und polyclonalen Antikörpern, wobei die erfindungsgemäßen Peptide als Antigene verwendet werden, insbesondere menschlichen oder tierischen Organismen zugeführt und die sich nach der Immunisierung bildenden Antikörper gewonnen beziehungsweise Antikörper bildende Zellen, zum Beispiel Milzzellen, zur Herstellung von monoclonale Antikörper bildenden Hybridomzellen erhalten werden. Die Erfindung betrifft also auch die Verwendung der erfindungsgemäßen Peptide zur Gewinnung von monoclonalen oder polyclonalen Antikörpern mittels an sich bekannter Verfahren, wobei es sich hierbei auch um rekombinante Verfahren, also zum Beispiel der Herstellung von Antikörpern in E. coli, handeln kann. Die gewonnenen Antikörper, bei welchen es sich also auch um humanisierte oder humane Antikörper handeln kann, können für Forschungszwecke, zum Beispiel als Forschungswerkzeuge, für therapeutische oder diagnostische Zwecke eingesetzt werden, insbesondere bei der Diagnose und Therapie der Alzheimerschen Krankheit. Die erfindungsgemäßen polyclonalen oder monoclonalen Antikörper können beispielsweise zur Analyse des Krankheitsverlaufes von zum Beispiel mit erfindungsgemäßen Peptiden behandelten, Patienten oder zur Isolierung und Identifizierung von weiteren therapeutisch wirksamen Peptiden dienen. Die erfindungsgemäßen Peptide erweisen sich im Rahmen ihres Einsatzes als Immunogen für die Herstellung von Antikörpern für diagnostische und therapeutische Zwecke aufgrund ihrer erheblich verbesserten Handhabbarkeit gegenüber den nativ-vorkommenden schwerlöslichen Analoga als besonders vorteilhaft. Die so hergestellten Antikörper können in einer Ausführungsform spezifisch die erfindungsgemäßen Peptide und in einer anderen Ausführungsform auch das nativ vorhandene Wildtyp β-AP, gegebenenfalls in aggregierter Form erkennen, so dass die erfindungsgemäßen Antikörper zum Beispiel zur Diagnose der Alzheimerschen Krankheit eingesetzt werden können. Die Erfindung betrifft auch Verfahren zur Immunisierung von menschlichen oder tierischen Organismen, wobei die erfindungsgemäßen Peptide menschlichen oder tierischen Organismen appliziert und eine Immunisierung gegen β-AP beziehungsweise dessen Derivate erreicht wird.

Die erfindungsgemäßen Peptide können in üblicher Weise chemisch synthetisiert und/oder modifiziert werden. Sie können auch durch rekombinante DNA-Technik hergestellt oder aus natürlichen Quellen isoliert und modifiziert werden.

Die Erfindung betrifft auch pharmazeutische Zusammensetzungen, enthaltend ein vorgenanntes cyclisches Peptid in pharmazeutisch wirksamer Menge und einen pharmazeutisch akzeptablen Träger. In einer Ausführungsform der vorliegenden Erfindung enthält die pharmazeutische Zusammensetzung mindestens ein cyclisches Peptid der vorliegenden Erfindung in einer prophylaktisch oder therapeutisch wirksamen Menge, die ausreicht, die Aggregation, insbesondere die Aggegationsrate und/oder die Menge gebildete Aggregate, nativer amyloider Peptide zu ändern, insbesondere zu inhibieren. In einer weiteren Ausführungsform der vorliegenden Erfindung weist die pharmazeutische Zusammensetzung der vorliegenden Erfindung mindestens ein cyclisches Peptid der vorliegenden Erfindung in einer prophylaktisch oder therapeutisch wirksamen Menge auf, die ausreicht, die neurotoxische Wirkung natürlicherweise vorkommender amyloider Peptide bzw. deren Aggregate zu ändern, insbesondere zu inhibieren. Die pharmazeutisch wirksame Menge hängt von verschiedenen Faktoren ab, wie dem Krankheitszustand, der Größe des Patienten, dem Gewicht des Patienten, der Menge an endogen vorhandenem amyloiden Peptid usw.

Pharmazeutisch verträgliche Träger sind zum Beispiel Lösungsmittel, Dispersionsmittel, Beschichtungen, Füllstoffe, antibakterielle und antifungale Agenzien, isotonische Agenzien usw. Gegebenenfalls können weitere Additive wie Farb- oder Geschmackstoffe, Emulgatoren, Bindemittel, Trennmittel etc. hinzugefügt werden.

In einer weiteren Ausführungsform der vorliegenden Erfindung kann vorgesehen sein, die pharmazeutische Zusammensetzung intravenös, oral, intraperitoneal, intraspinal, intrazerebral oder intramuskulär zu verabreichen. Es kann vorgesehen sein, die Zusammensetzung in Form einer sterilen wässrigen Lösung oder Dispersion oder eines Pulvers zu verabreichen.

Gegebenenfalls kann auch vorgesehen sein, weitere medizinisch wirksame Substanzen zusammen mit dem cyclischen Peptid der vorliegenden Erfindung in der pharmazeutischen Zusammensetzung zu verwenden. Es kann vorgesehen sein, weitere Substanzen in der pharmazeutischen Zusammensetzung einzusetzen, die zum Beispiel dem Transport im Zielorganismus, beispielsweise durch die Blut-Hirn-Schranke, dienen.

In einer weiteren bevorzugten Ausführungsform der vorliegenden Erfindung betrifft diese diagnostische Zusammensetzungen, enthaltend mindestens ein cyclisches Peptid der vorliegenden Erfindung, welches vorzugsweise mit einem Detektionsmarker versehen ist. Ein derartiger Detektionsmarker kann eine radioaktive Markierung wie zum Beispiel radioaktives Jod oder Technetium sein. Der Detektionsmarker kann jedoch auch ein Fluoreszenzmarker, Lumineszenz- oder Enzymmarker, oder ein Spin-Label sein.

In einer weiteren bevorzugten Ausführungsform betrifft die Erfindung ein Verfahren zum Nachweis, insbesondere Screenen, Absuchen oder Detektieren, amyloidogener Peptide, insbesondere β-AP oder Derivaten davon, von Oligomeren aus amyloidogenen Peptiden oder Aggregaten aus amyloidogenen Peptiden sowie von Antikörpern gegen die sogenannten Materilien in einer biologischen Probe oder einem tierischen oder menschlichen Organismus, wobei mindestens ein, vorzugsweise mit einem Detektionsmarker versehenes, cyclisches Peptid der vorliegenden Erfindung oder ein mit einem Detektionsmarker versehener Antikörper der vorliegenden Erfindung als Sonde mit einer zu untersuchenden Probe,vorzugsweise in einer Flüssigkeit, in Kontakt gebracht und eine Assoziierung des Peptides mit amyloidogenen Peptiden, Oligomeren davon, Aggregaten davon oder Antikörpern dagegen qualitativ oder/und quantitativ nachgewiesen wird. In einer Ausführungsform der vorliegenden Erfindung kann dieses Verfahren in vitro und in einer anderen Ausführungsform in vivo durchgeführt werden. Der Begriff in-Kontakt-bringen erfasst daher die Inkubation des cyclischen Peptides mit einer Probe in vitro oder das Einbringen des cyclischen Peptides zu einem Ort in vivo, wo zum Beispiel natürliches amyloides Peptid oder deren Aggregate vorhanden ist. Selbstverständlich ist es auch vorgesehen, dieses Verfahren in Gegenwart mindestens einer weiteren Testsubstanz, zum Beispiel von nativem β-AP, anderer Peptidvarianten, potentiellen Pharmazeutika, potentiellen Diagnostika oder Markersubstanzen in Form zum Beispiel kompetitiver Testverfahren durchzuführen, wobei die Wirkung dieser weiteren Testsubstanz auf das Aggregationsverhalten von natürlichen β-AP oder/und erfindungsgemäßen Peptiden nachgewiesen wird.

Erfindungsgemäß kann vorgesehen sein, die cyclischen Peptide in immobilisierter Form zu verwenden. So kann in bevorzugter Weise vorgesehen sein, das cyclische Peptid mit weiteren funktionellen Gruppen zu versehen, die eine Immobilisierung an festen Trägern ermöglichen. Die Immobilisierung kann beispielsweise bei der Anwendung der erfindungsgemäßen Peptide in Antikörper-basierten Test-, Screening- und Analyseverfahren zum Beispiel zur Identifizierung von β-AP oder β-AP-Derivat-spezifischen Antikörpern vorgenommen werden. Die Immobilisierung kann auch für präparative Verfahren in der Forschung und Therapie eingesetzt werden, wie für Affinitätsmatrices zur Bindung, Isolierung und Abreicherung von Amyloiden aus verschiedenen Flüssigkeiten.

Die Erfindung betrifft auch die Verwendung mindestens eines cyclischen Peptids der vorliegenden Erfindung zur Herstellung eines Arzneimittels für die Behandlung von durch Amyloidbildung gekennzeichneten Krankheiten, das heißt Amyloidosen, insbesondere primäre, sekundäre, vererbliche oder/und isolierte Amyloidosen wie die Alzheimersche Krankheit, insbesondere sporadische oder familiengebundene Alzheimersche Krankheit.

Die Erfindung betrifft also auch Verfahren zur Diagnose und/oder Therapie, zum Beispiel Prophylaxe von Amyloidosen, gemäß der mindestens ein cyclisches Peptid der vorliegenden Erfindung einem menschlichen oder tierischen Körper oder einem isolierten Bestandteil davon in einer pharmazeutisch oder diagnostisch wirksamen Menge appliziert und im Falle des diagnostischen Einsatzes eine Bindung an amyloide Strukturen nachgewiesen wird.

Die Erfindung betrifft auch Verfahren zur Veränderung der Aggregation amyloidogener Peptide, Oligomeren oder Aggregaten davon oder zur Inhibierung der Zytotoxizität von amyloidogenen Peptiden, Oligomeren oder Aggregaten davon, wobei Peptide der vorliegenden Erfindung in vivo oder in vitro mit den amyloidogenen Peptiden, Oligomeren oder Aggregaten davon in Kontakt gebracht werden und das Aggregationsverhalten der amyloidogenen Peptide, Oligomere oder Aggregate davon geändert wird, insbesondere die Aggregation reduziert oder inhibiert wird. Eine Amyloidose im Sinne der vorliegenden Erfindung ist insbesondere die Alzheimersche Krankheit.

Insbesondere betrifft die Erfindung ein Verfahren zur Modifizierung, vorzugsweise Verhinderung, der Aggregatbildung von in einer Flüssigkeit enthaltenem amyloidem β-AP, wobei ein Peptid der vorliegenden Erfindung mit der Flüssigkeit in Kontakt gebracht und inkubiert wird. Dieses Verfahren kann auch zur Abreicherung beziehungsweise Entfernung von Amyloid aus Flüssigkeit, zum Beispiel Körperflüssigkeiten, dienen.

Die vorliegende Erfindung betrifft auch Kits, umfassend die erfindungsgemäßen cyclischen Peptide und/oder dagegen gerichtete monoclonale oder polyclonale Antikörper, wobei diese Agenzien gegebenenfalls markiert sein können.

Weitere vorteilhafte Ausgestaltungen der Erfindung ergeben sich aus den Unteransprüchen.

Die Erfindung wird anhand der Beispiele, des Sequenzprotokolls und der dazugehörigen Figuren näher erläutert.

Die Figuren 1 und 2 zeigen elektronenmikroskopische Aufnahmen des Fibrillierungsverhaltens von cβ-AP128 (1A), β-AP(1-28) (1B), [Lys¹⁷, Asp²¹] β-AP (1-28) (lineares Kontrollpeptid) (1C) sowie β-AP(1-28) (2A) und einer 1:1-Mischung von β-AP(1-28) und cβ-AP128 (2B).

Figur 3 zeigt schematisch die Struktur eines erfindungsgemäßen eine intramolekulare Brücke aufweisenden Peptids sowie der linearen Kontrollpeptide.

Das zu der vorliegenden Lehre gehörige Sequenzprotokoll zeigt:
SEQ ID Nr. 1 die Wildtyp-Aminosäuresequenz von β-AP (1-43),
SEQ ID Nr. 2-19 die erfindungsgemäßen Aminosäuresequenzen, wobei eine intramolekulare Brückenbildung jeweils zwischen den Seitenketten der Aminosäuren 17 und 21 vorliegt.

### Beispiel 1

### Herstellung, Reinigung und Charakterisierung von cβ-AP128 und Control-β-AP(1-28)

Die Peptide wurden nach gängigen Methoden der Festphasenpeptidsynthese unter Verwendung der Boc/Bzl-Synthesestrategie an PAM-Harz hergestellt (Barany, G., Kneib-Cordonier, N. & Mullen, D. G. (1987) J. Pept. Res. 30, 705-739), mittels HF/Anisol (10:1) vom Harz abgespalten und durch präparative HPLC über eine C18-Säule gereinigt (Kapurniotu & Taylor, (1995) J. Med. Chem. 38, 836-847). Für die Synthese von cβ-AP128 wurden die Seitenketten von Lys¹⁷ und Asp²¹ mittels der Fmoc- beziehungsweise Fluorenylmethylester (OFm)-Schutzgruppen geschützt, welche dann selektiv mittels 20% Piperidin/DMF vor Bildung der Laktambrücke abgespalten wurden (Kapurniotu & Taylor, J. Med. Chem. 38, 836-847 (1995)). Die Seitenketten-zu-Seitenketten-Zyklisierung wurde mit Benzotriazol-1-yloxy-tris-(dimethylamino) phosphonium-hexafluorophosphat (BOP) durchgeführt und mit dem Kaisertest auf Vollständigkeit überprüft (Kapurniotu & Taylor (1995) J. Med. Chem. 38, 836-847)). Um eine vollständige Zyklisierungsreaktion zu erhalten, wurde die Zyklisierungsreaktion in Mischungen aus DMF, NMP und DMSO 4-mal (die Dauer einer Zyklisierung betrug um die 20 h) durchgeführt. Die Massen der mittels HPLC gereinigten Produkte wurden durch MALDI-MS bestimmt und ihre Reinheit durch analytische HPLC verifiziert.

### Beispiel 2

### cßAP128: Aggregation, Testung der Fibrillenbildung, Testung der inhibitorischen Wirkung auf die Aggregation von β-AP(1-28).

### Physikochemische Eigenschaften von cβ-AP128 und Testung der Fibrillenbildung

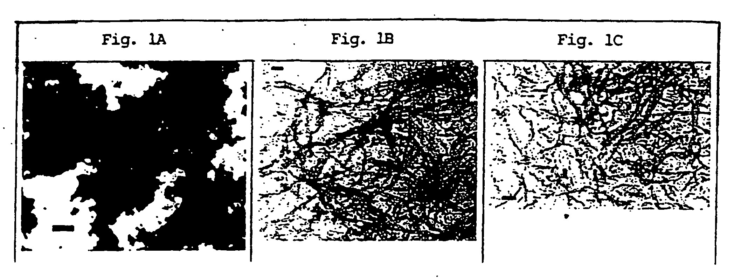

Die physikochemischen Eigenschaften von cβ-AP wurden durch FT-IR (Fourier transformierte Infrarot-Spektroskopie) und CD (Circulardichroismus-Spektropolarimetrie) untersucht. EM (Elektronenmikroskopie) und AFM (Rasterkraftmikroskopie) wurden verwendet, um die Fibrilleneigenschaften von cβ-AP128 zu testen. Für die EM-Studien wurden zunächst 450 µM Lösungen von cβ-AP128 oder β-AP(1-28) oder [Lys¹⁷, Asp²¹]β-AP(1-28) in 10 mM Phosphatpuffer, 150 mM NaCl und 30% Acetonitril, pH 5,5, mehrere Tage lang inkubiert und auf Fibrillenbildung untersucht. Diese Studien zeigten, dass hochkonzentrierte Lösungen vom cβ-AP128 mehrere Tage lang (mehr als 30) keine Fibrillen bilden (Abb. 1A); dies ist im Gegensatz zu den Kontrollpeptiden β-AP(1-28) (nativ vorkommende Sequenz) und [Lys¹⁷, Asp²¹]β-AP(1-28)(lineares Kontrollpeptid), welche nach drei Tagen Inkubation vollständig fibrilliert sind (Abb. 1B, 1C).

FT-IR der obigen Lösungen/Suspensionen zeigte, dass die eingeführte Verbrückung in cβ-AP128 die β-Faltblatt Konformation, welche natives βAP und das Kontrollpeptid [Lys¹⁷, Asp²¹]β-AP(1-28) nach Alterung einnehmen, unterbindet und somit die Aggregation und Fibrillenbildung inhibiert.

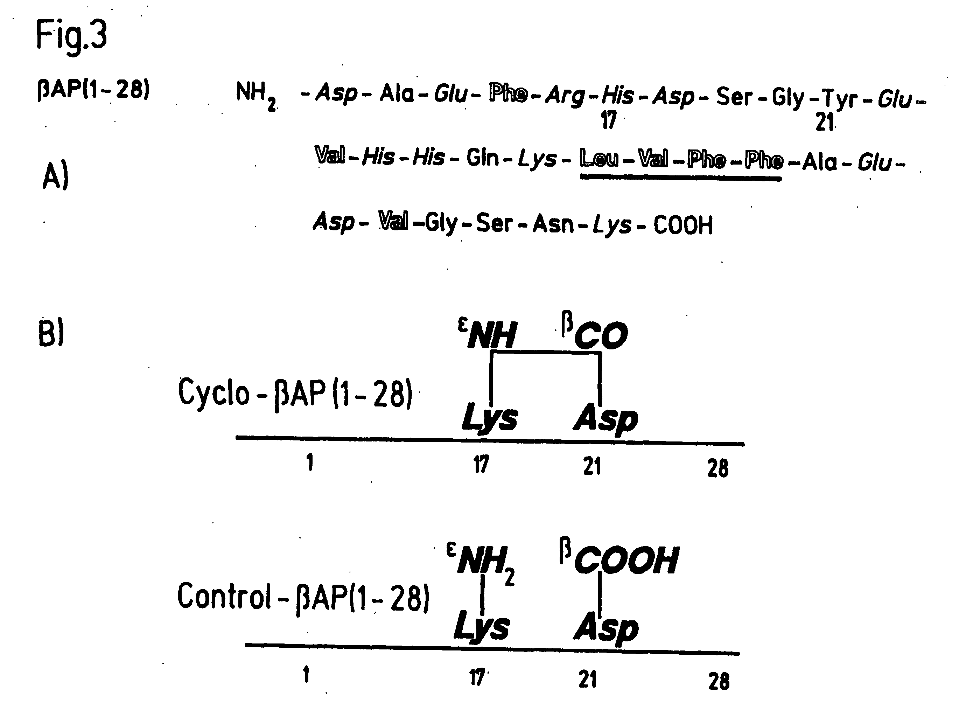

Die Figur 3 zeigt in A) die native Aminosäuresequenz des β-AP-Fragmentes β-AP(1-28). Die Positionen 17 bis 20 sind hervorgehoben, um ihre wichtige Rolle bei der Assoziation und Fibrillenbildung darzustellen. Im oberen Teil von Figur 3B wird schematisch die Struktur des erfindungsgemäßen β-AP(1-28)-Derivats Cyclo^{17,21} [Lys¹⁷, Asp²¹] β-AP(1-28) abgekürzt als Cyclo-β-AP(1-28) oder bezeichnet als cβ-AP128 mit der intramolekularen Brücke zwischen den Seitenketten von Lys¹⁷ und Asp²¹, insbesondere der ε-Aminogruppe von Lys und der β-Carboxylgruppe von Asp dargestellt. Es handelt sich bei diesem Beispiel also um eine intramolekulare Brücke, wobei ohne Zwischenschaltung eines Linkers oder Spacers die Seitenketten der Aminosäuren an den Positionen 17 und 21 durch eine Lactambindung kovalent verknüpft werden. Im unteren Teil von Figur 3B wird das Kontrollpeptid Kontroll-β-AP(1-28), also [Lys¹⁷, Asp²¹]β-AP(1-28), dargestellt. Die Figur verdeutlicht, dass durch den Austausch des Leucin an Position 17 durch Lysin und von Alanin an Position 21 durch Asparaginsäure Analoga erhalten werden, die sich vom nativen β-AP(1-28)-Peptid unterscheiden und die durch kovalente Bindung der Seitenketten dieser Substituenten, also durch die eingeführten Lactambrücken zwischen der ε-Aminogruppe und der β-Carboxygruppe zur erfindungsgemäßen Struktur führen.

### Beispiel 3

### Inhibition der Aggregation und Fibrillenbildung von β-AP(1-28) oder [Lys¹⁷, Asp²¹]β-AP(1-28) durch cβ-AP128

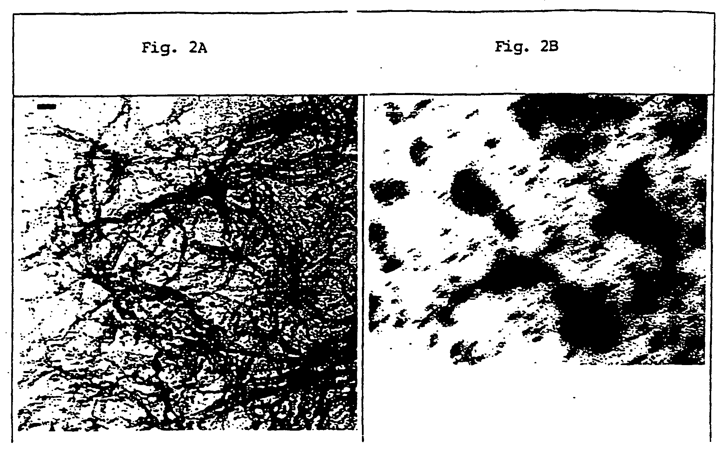

Der Einfluss von cβ-AP128 auf die Aggregations- und Fibrillenbildung von β-AP(1-28) wurde durch CD und EM getestet. Eine Lösung von 450 µM β-AP(1-28) und cβ-AP128 in 10 mM Phosphatpuffer, 150 mM NaCl und 30 % Acetonitril, pH 5,5, wurde mehrere Tage lang inkubiert und durch EM auf Fibrillierung getestet (Abb. 2A und 2B). CD zeigte keine Veränderungen der CD-Spektra für mehr als 1 Monat in diesen Mischungen, obwohl β-AP allein oder [Lys¹⁷, Asp²¹] β-AP(1-28) allein in weniger als 10 Stunden β-Faltblätter gebildet haben und anschließend als unlösliches Amyloid auspräzipitierten.

## Patentansprüche

1. Peptid mit der biologischen Aktivität eines Modulators der Amyloidogenese mit einer Aminosäuresequenz, ausgewählt aus der Gruppe der Sequenzen bestehend aus SEQ ID Nr. 2 bis SEQ ID Nr. 19, wobei das Peptid mindestens eine intramolekulare Brücke aufweist, die durch kovalente Bindung zwischen den Seitenketten zumindest der Aminosäuren an den Positionen 17 und 21 des Peptids gebildet wird.

2. Peptid nach Anspruch 1, wobei der Modulator ein Inhibitor ist.

3. Peptid nach einem der vorhergehenden Ansprüche, wobei die Seitenketten der an der Brückenbildung beteiligten Aminosäuren des Peptids direkt oder über ein Verbindungsmolekül kovalent miteinander verbunden sind.

4. Peptid nach einem der vorhergehenden Ansprüche, wobei das Verbindungsmolekül eine Aminocarboxy-, eine Diamino- oder Dicarboxyverbindung ist.

5. Peptid nach einem der vorhergehenden Ansprüche, wobei das Peptid mindestens eine weitere funktionelle Gruppe ausgewählt aus der Gruppe bestehend aus Alkylgruppe, funktionalisierter Alkylgruppe, aromatischer Gruppe, Oligopeptide, Polypeptide, Glycogruppe und Lipidgruppe aufweist.

6. Peptid nach einem der vorhergehenden Ansprüche, wobei das Peptid auf einem Träger immobilisiert ist.

7. Antikörper, der spezifisch ein Peptid nach einem der Ansprüche 1 bis 6 erkennt.

8. Antikörper nach Anspruch 7, der ein monoclonaler oder polyclonaler Antikörper ist.

9. Pharmazeutische Zusammensetzung, enthaltend ein Peptid nach einem der Ansprüche 1 bis 6 und einen pharmazeutisch verträglichen Träger.

10. Diagnostische Zusammensetzung, enthaltend ein mit einem Detektionsmarker versehenes Peptid nach einem der Ansprüche 1 bis 6 oder einen Antikörper nach Anspruch 7 oder 8.

11. Diagnostische Zusammensetzung nach Anspruch 10, wobei der Detektionsmarker ein radioaktiver Marker, ein Fluoreszenzmarker, ein Enzymmarker, ein Luminenzenzmarker oder ein Spin-Label ist.

12. Verfahren zum qualitativen und/oder quantitativen Nachweis von amyloidogenem β-AP oder dessen Aggregaten, wobei ein mit einem Detektionsmarker versehenes Peptid nach einem der Ansprüche 1 bis 6 oder ein mit einem Detektionsmarker versehender Antikörper nach einem der Ansprüche 7 oder 8 als Sonde mit einer zu untersuchenden Probe in Kontakt gebracht und eine Bindung des Peptids mit gegebenenfalls vorhandenen amyloidogenen β-AP-Peptiden oder dessen Aggregaten nachgewiesen wird.

13. In vitro-Verfahren zur Modifizierung, insbesondere Verhinderung der Aggregatbildung von in einer Flüssigkeit enthaltendem amyloidogenem β-AP, wobei ein Peptid nach einem der Ansprüche 1 bis 6 mit der Flüssigkeit in Kontakt gebracht und inkubiert wird.

14. Verwendung eines Peptides nach einem der Ansprüche 1 bis 6 oder/und eines Antikörpers nach einem der Ansprüche 7 oder 8 zur Herstellung eines Arzneimittels für die Prophylaxe und/oder Behandlung von Amyloidosen, insbesondere der Alzheimerschen Krankheit.

15. Verwendung eines Peptides nach einem der Ansprüche 1 bis 6 als ein verbessert handhabbares Immunogen für die Herstellung von Antikörpern für diagnostische, therapeutische oder Forschungs-Zwecke

16. In vitro-Verfahren nach Anspruch 13 zur präparativen Abreicherung von Amyloid aus Flüssigkeiten, insbesondere Körperflüssigkeiten.

## Claims

1. Peptide with the biological activity of a modulator of amyloidogenesis with an amino acid sequence, selected from the group of sequences comprising SEQ ID No. 2 to SEQ ID No. 19, wherein the peptide has at least one intramolecular bridge, which is formed by covalent bonding between the side chains of at least the amino acids onto positions 17 and 21 of the peptide.

2. Peptide according to Claim 1, wherein the modulator is an inhibitor.

3. Peptide according to one of the preceding claims, wherein the side chains of the amino acids of the peptide taking part in the bridge formation are covalently bonded to one another directly or via a connector molecule.

4. Peptide according to one of the preceding claims, wherein the connector molecule is an amino carboxy, diamino- or dicarboxy compound.

5. Peptide according to one of the preceding claims, wherein the peptide has a further functional group selected from the group comprising the alkyl group, functionalised alkyl group, aromatic group, oligopeptides, polypeptides, glyco group and lipid group.

6. Peptide according to one of the preceding claims, wherein the peptide is immobilised on a carrier.

7. Antibody, which specifically recognises a peptide according to one of Claims 1 to 6.

8. Antibody according to Claim 7, which is a monoclonal or polyclonal antibody.

9. Pharmaceutical composition containing a peptide according to one of Claims 1 to 6 and a pharmaceutically compatible carrier.

10. Diagnostic composition containing either a peptide according to one of Claims 1 to 6 provided with a detection marker or an antibody according to Claim 7 or 8.

11. Diagnostic composition according to Claim 10, wherein the detection marker is a radioactive marker, a fluorescent marker, an enzyme marker, a luminescent marker or a spin label.

12. Method for the qualitative and/or quantitative identification of amyloidogenic β-AP or its aggregates, wherein a peptide according to one of Claims 1 to 6 provided with a detection marker or an antibody according to one of Claims 7 or 8 is brought as a probe into contact with a sample to be examined and a bond of the peptide with possibly present amyloidogenic β-AP peptides or their aggregates is identified.

13. In vitro method for modification, in particular prevention, of the aggregate formation of amyloidogenic β-AP contained in a liquid, wherein a peptide according to one of Claims 1 to 6 is brought into contact with the liquid and incubated.

14. Use of a peptide according to one of Claims 1 to 6 or/and an antibody according to one of Claims 7 or 8 for the production of a medication for the prophylaxis and/or treatment of amyloidoses, in particular of Alzheimer's disease.

15. Use of a peptide according to one of Claims 1 to 6 as a more readily usable immunogen for the production of antibodies for diagnostic, therapeutic or research purposes.

16. In vitro method according to Claim 13 for the preparative removal of amyloid from liquids, in particular body fluids.

## Revendications

1. Peptide ayant l'activité biologique d'un modulateur de l'amyloïdogenèse et ayant une séquence d'acides aminés choisie dans le groupe des séquences constituées de SEQ ID N°2 à SEQ ID N°19, le peptide présentant au moins un pont intramoléculaire formé par liaison covalente entre les chaînes latérales au moins des acides aminés situés sur les positions 17 et 21 du peptide.

2. Peptide selon la revendication 1,
**caractérisé en ce que**
le modulateur est un inhibiteur.

3. Peptide selon l'une des revendications précédentes,
**caractérisé en ce que**
les chaînes latérales des acides aminés du peptide participant à la formation d'un pont sont reliées entre elles de façon covalente, soit directement, soit par l'intermédiaire d'une molécule de liaison.

4. Peptide selon l'une des revendications précédentes,
**caractérisé en ce que**
la molécule de liaison est un composé aminocarboxy, diamino ou dicarboxy.

5. Peptide selon l'une des revendications précédentes,
**caractérisé en ce que**
le peptide présente au moins un autre groupe fonctionnel choisi dans la catégorie comprenant un groupe alkyle, un groupe alkyle fonctionnalisé, un groupe aromatique, un oligopeptide, un polypeptide, un groupe glyco et un groupe lipide.

6. Peptide selon l'une des revendications précédentes,
**caractérisé en ce que**
le peptide est immobilisé sur un support.

7. Anticorps qui reconnaît spécifiquement un peptide selon l'une des revendications 1 à 6.

8. Anticorps selon la revendication 7, qui est un anticorps monoclonal ou polyclonal.

9. Composition pharmaceutique contenant un peptide selon l'une des revendications 1 à 6 et un support pharmaceutiquement acceptable.

10. Composition de diagnostic contenant un peptide muni d'un marqueur de détection selon l'une des revendications 1 à 6 ou un anticorps selon la revendication 7 ou 8.

11. Composition de diagnostic selon la revendication 10,
**caractérisée en ce que**
le marqueur de détection est un marqueur radioactif, un marqueur de fluorescence, un marqueur enzymatique, un marqueur de luminescence ou un marqueur de spin.

12. Procédé de mise en évidence qualitative et/ou quantitative de β-AP amyloïdogène ou de ses agrégats, selon lequel on met en contact un peptide muni d'un marqueur de détection selon l'une des revendications 1 à 6 ou un anticorps muni d'un marqueur de détection selon l'une des revendications 7 ou 8 comme sonde, avec un échantillon à examiner, et on met en évidence une liaison du peptide avec des β-AP-peptides amyloïdogènes ou leurs agrégats éventuellement présents.

13. Procédé in vitro pour modifier, en particulier pour empêcher, la formation d'agrégats de β-AP amyloïdogène contenu dans un liquide, selon lequel on met en contact un peptide selon l'une des revendications 1 à 6 avec le liquide et on le fait incuber.

14. Utilisation d'un peptide selon l'une des revendications 1 à 6 et/ou d'un anticorps selon l'une des revendications 7 ou 8, pour la production d'un médicament pour la prophylaxie et/ ou le traitement des amyloïdoses, en particulier de la maladie d'Alzheimer.

15. Utilisation d'un peptide selon l'une des revendications 1 à 6, comme immunogène manipulable de façon améliorée pour la production d'anticorps dans des buts de diagnostic, de traitement ou de recherche.

16. Procédé in vitro selon la revendication 13, pour l'extraction préparative d'amyloïde à partir de liquides, en particulier de liquides corporels.
